# EUROPEAN PATENT APPLICATION

(11) **EP 3 632 299 A1**
(43) Date of publication of application: **08.04.2020**
(21) Application number: 18199010.2
(22) Date of filing: 05.10.2018
(51) Int. Cl.: A61B 5/00, A61B 5/0476

(54) **METHOD AND SYSTEM FOR MONITORING A SUBJECT**

(71) Applicant: Epihunter NV, 3500 Hasselt (BE)
(72) Inventor: LOECKX, Dirk, 3010 Leuven (BE); BUCKINX, Tim Bert, 3511 Hasselt (BE)
(74) Representative: IPLodge bvba

(57) **Abstract**

The invention pertains to a method for monitoring a subject, the method comprising: capturing (1010) high-bandwidth data pertaining to said subject; cyclically storing (1020) a recent portion of said high-bandwidth data in a first memory; monitoring (1030) one or more physiological signals of said subject; analyzing (1040) said monitored physiological signals to allow detection of one or more target events; upon detection of a target event: copying (1051) a then recent portion of said high-bandwidth data from said first memory to a second memory; and storing (1052) high-bandwidth captured in a predetermined period subsequent to said detection in said second memory. The invention also pertains to a computer program product and to a system.

## Description

### Field of the Invention

The present invention relates to the field of medical monitoring methods and equipment, in particular for the monitoring of subjects suffering from intermittent neurological episodes such as epileptic seizures.

### Background

European patent application no. EP 2 555 675 A1 in the name of Flint Hills Scientific LLC, entitled "Responsiveness testing of a patient having brain changes", discloses a method of determining a responsiveness of a patient having brain state changes, comprising receiving an indication of a triggering event; administering to the patient, in response to the indication, a test of responsiveness; determining, based upon a result of the test, at least one responsiveness parameter selected from the group consisting of (i) a time of occurrence of a change in the patient's responsiveness, (ii) a duration of a change in the patient's responsiveness; (iii) a magnitude of a change in the patient's responsiveness, (iv) a time interval from the indication of event occurrence to a change in the patient's responsiveness, (v) a type of change in the patient's responsiveness, (vi) an estimation of a seizure severity; (vii) a classification of a seizure into clinical or subclinical; (viii) a classification of a clinical seizure into simple partial, complex partial, or generalized; (ix) an assessment of efficacy of a therapy for the patient's medical condition; (x) an assessment of the state of the disease and formulation of a prognosis for the patient; (xi) an estimation of a risk of injury or death for the patient; and (xii) two or more thereof.

The method of EP 2 555 675 A1 is based on the premise that observing the subject's reactivity after the detection of a brain state change is sufficient to classify a brain state change. The inventors of the present invention have found that this is not always the case. If the assessment or observation of the subject as provided in EP 2 555 675 A1 were simply applied on a continuous basis without reference to a brain state change detection, the method would become impracticable outside the context of a professional medical environment.

### Summary

According to an aspect of the present invention, there is provided a method for monitoring a subject, the method comprising: capturing high-bandwidth data pertaining to said subject; cyclically storing a recent portion of said high-bandwidth data in a first memory; monitoring one or more physiological signals of said subject; analyzing said monitored physiological signals to allow detection of one or more target events; upon detection of a target event: copying a then recent portion of said high-bandwidth data from said first memory to a second memory; and storing high-bandwidth data captured in a predetermined period subsequent to said detection in said second memory.

The term "target events", as used herein, refers to the set of physiological conditions of which observation is intended. This set may in particular include neurological events such as epileptic seizures (in particular absence seizures), episodic paroxysmal sympathetic hyperactivity (sympathetic storm), and the like.

The term "high-bandwidth data", as used herein, refers to data representing signals that, when stored in digital form, consist of significant amounts of digital data per unit of time. In particular, a signal having a bandwidth that would consume a significant portion of the uplink bandwidth of the device capturing and temporarily storing the data may be considered as "high-bandwidth data" for the purpose of the present invention. The invention is particularly useful in situation where the bandwidth of the "high-bandwidth data" exceeds the uplink bandwidth of the device capturing and temporarily storing the data. Depending on the device, the term may for example and without limitation include data streams accruing at rates in excess of 100 kbps, in excess of 500 kbps, or in excess of 1000 kbps, (for example, MPEG4-compressed VGA-resolution video at 30 fps represents a high-bandwidth data stream of approximately 1500 kbps; further for example, MPEG4-comressed HD-resolution video at 30 fps represents a high-bandwidth data stream of approximately 4000 kbps).

The present invention is based *inter alia* on the insight of the inventors that information pertaining to the subject in the moments prior to the physiological detection of certain target events can provide valuable insights into the subject's condition, possibly leading to improved management of the condition or better-tailored responses by caregivers.

The present invention is further based on the insight of the inventors that it is inefficient to store or transmit high-bandwidth data spanning long periods of time, just in case one or more target events might occur during the monitored periods. This is of particular concern when the monitoring and storage are to be performed by a device with limited storage capacity, such as a mobile device, a wearable device, an embedded device, an implant, or the like. In addition, storage of certain types of data (for example, video monitoring data) covering extended periods of time may raise privacy concerns.

The present invention resolves these issues by providing a limited, temporary, cyclical storage of all captured high-bandwidth data, of which only the parts that are relevant to a target event are copied to a more permanent storage for later analysis by the subject, their caregivers, medical professionals, researchers, and the like. While this approach is known from the field of "dashcams" (see for example US patent application publication no. US 2016/0381292 A1 and US patent no. 9,307,217 B1), it has never been applied to the monitoring of subjects with medical conditions in conjunction with physiological sensor data, the analysis of which provides the trigger for the permanent storage of preceding video data.

In an embodiment of the method according to the present invention, the high-bandwidth data comprises video images of the subject.

This embodiment is based on the insight of the inventors that audiovisual information pertaining to the subject in the moments prior to the physiological detection of certain target events can provide valuable insights into the subject's condition, possibly leading to improved management of the condition or better-tailored responses by caregivers. The storage and privacy concerns discussed above apply in particular to audiovisual data streams comprising video images of the subject.

In an embodiment of the method according to the present invention, the high-bandwidth data comprises high-resolution EEG signals.

Electroencephalography (EEG) signals, capture just prior to the onset of a target event, are particularly useful for better understanding the triggers and circumstances of the target event when the target event is a neurological condition or associated with a neurological condition, such as epilepsy.

In an embodiment of the method according to the present invention, the first memory is a circular buffer.

This is a particularly efficient implementation for the cyclical storage of data, wherein the oldest data is systematically overwritten by more recent data, thus ensuring that the most recent data is in memory at any given time.

In an embodiment of the method according to the present invention, said storing of said high-bandwidth data captured in said predetermined period subsequent to said detection comprises copying a further portion of said high-bandwidth data from said first memory to said second memory.

As it is typically desirable to keep the high-bandwidth data captured during the target event, which may last from a few seconds to several minutes, it is particularly advantageous to provide a sufficiently large first memory to hold the high-bandwidth data pertaining to both the pre-event period of interest and the actual event. The transfer of the high-bandwidth data to more permanent storage may then consist of copying a single contiguous section of high-bandwidth data from the first memory to the second memory.

In an embodiment of the method according to the present invention, said first memory is comprised in a mobile device and said capturing of said high-bandwidth data is performed using a sensor associated with said mobile device.

It is an advantage of this embodiment that it can be applied in a manner that is minimally invasive in the subject's daily routine. A mobile device may be positioned in a fixed position in the subject's area of presence (e.g. a dwelling, a place of employment, a classroom, a hospital room, etc.), whereby the sensor associated with the mobile device is used for capturing the high-bandwidth data and a memory inside the mobile device serves as the first memory.

The sensor associated with the mobile device may be integrated in the mobile device, or communicating with the mobile device through a suitable network connection (e.g., a Personal Area Network such as Bluetooth or Bluetooth LE, USB, and the like).

In a particular embodiment, the second memory is comprised in the mobile device.

In this embodiment, no permanent connection between the mobile device and a network-based storage is necessary to transfer the video clips of interest, as the latter are also stored on the mobile device.

In another particular embodiment, the second memory is comprised in an apparatus separate from the mobile device, the mobile device being configured to transmit data to the apparatus via a data communication network.

This embodiment relies on a network-based storage, such as "cloud storage" or network-attached storage solutions, to overcome the capacity limits of the storage components comprised in a mobile device. An additional advantage of this embodiment is that the stored video data can easily be made available to third parties for further processing and analysis.

In a particular embodiment, the high-bandwidth data comprises video images and the sensor associated with the mobile device is a camera integrated in said mobile device.

This is a particularly advantageous way to capture video images of said subject as the high-bandwidth data.

In an embodiment of the method according to the present invention, the one or more physiological signals include one or more of the group of EEG, ECG, body temperature, movement, skin conductivity, heart rate, oxygen saturation.

It has been found that subsets of these physiological signals are capable of demonstrating or predicting the occurrence of certain target events.

In an embodiment of the method according to the present invention, said one or more target events include epileptic seizures.

For the specific case of epilepsy, the occurrence of seizures, and in particular absence seizures, is accurately detected by means of EEG signals.

In an embodiment of the method according to the present invention, said analyzing comprises applying a deep-learning algorithm.

Deep-learning algorithms have been proven to be particularly adequate at detecting patterns in large sets of input signals, to accurately identify the occurrence of a target event.

According to an aspect of the present invention, there is provided a computer program product comprising code means configured to cause a processor, when executed, to perform the steps of the method as described above.

According to an aspect of the present invention, there is provided a system for monitoring a subject, the system comprising: a first sensor arranged for capturing high-bandwidth data pertaining to said subject; a first memory adapted to cyclically store a recent portion of said captured high-bandwidth data; a second memory adapted for long-term storage of high-bandwidth data; second sensors adapted to monitor one or more physiological signals of said subject; and processing means configured to: analyze said monitored physiological signals to allow detection of one or more target events; and upon detection of a target event: copy a then recent portion of said high-bandwidth data from said first memory to said second memory; and store high-bandwidth data captured in a predetermined period subsequent to said detection in said second memory.

In an embodiment of the system according to the present invention, said high-bandwidth data comprises video images and said sensor arranged for capturing high-bandwidth data pertaining to said subject comprises a camera arranged for capturing video images of said subject.

In an embodiment of the system according to the present invention, at least said camera, said first memory, and said processing means are comprised in a mobile device.

The technical effects and advantages of embodiments of the computer program product and the system according to the present invention correspond, *mutatis mutandis,* to those of the corresponding embodiments of the method according to the present invention.

### Brief Description of the Figures

These and other features and advantages of embodiments of the present invention will now be described with reference to the accompanying drawings, in which:
- Figure 1 provides a flow chart of an embodiment of the method according to the present invention;
- Figure 2 schematically illustrates an embodiment of the system according to the present invention;
- Figure 3 is a schematic timing diagram further illustrating the operation of the present invention.

### Description of Embodiments

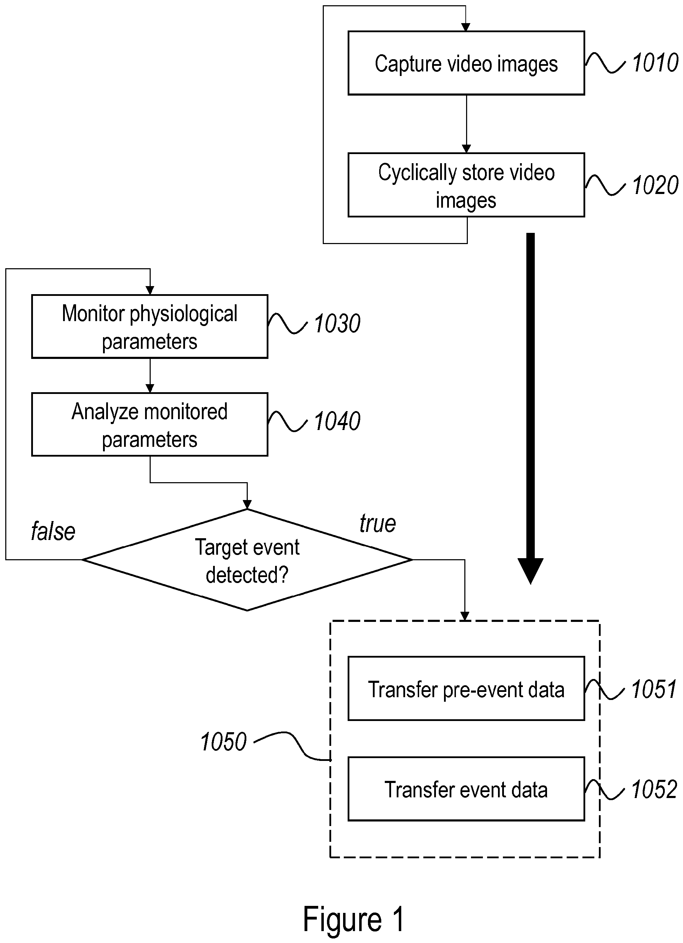

Figure 1 provides a flow chart of an embodiment of the method according to the present invention.

Throughout the application of the method according to the present invention, high-bandwidth data pertaining to the subject are captured. Without loss of generality, the figures and the present description will use the example of capturing video images of the subject **1010** for illustration purposes.

A recent portion of these video images is cyclically stored **1020** in a first memory, such as a circular buffer, in such a way that the most recent bit of video data is present in the first memory at any given time. The capturing **1010** and cyclic storage **1020** are preferably performed by a mobile device, such as a PDA, smart phone, or tablet. The mobile device may be installed in a cradle that ensures a continuous power supply and/or network connectivity.

The recent portion of the video images that is stored **1020** in the first memory preferably spans at least 10 seconds, more preferably at least 30 seconds, and most preferably at least 60 seconds.

In parallel, one or more physiological signals of the subject are being monitored **1030.** Without limitation, these physiological signals may include EEG, ECG, body temperature, movement, skin conductivity, heart rate, and oxygen saturation.

The physiological signals are provided by sensor devices, which are preferably non-invasive, or minimally invasive. For example, absence seizures are advantageously detected by analyzing EEG signals, which may be captured by a non-invasive headset worn by the subject, which may be shaped, without limitation, as a hat, cap, head-strap, visor, or the like. Heart rate, oxygen saturation, and body temperature can be sensed by means of optical sensors, which may be placed in contact with the subject's body in the form of wristbands, finger pinching apparatus, chest straps, and the like. The sensor devices may relay their signals to the above mentioned mobile device via a wireless personal area network interface (e.g. Bluetooth or Bluetooth LE) or a wireless local area network interface (e.g. IEEE Std 802.11 "Wi-Fi").

The physiological signals are analyzed **1040** to allow detection of one or more target events, for example by applying a deep-learning algorithm. The analyzing **1040** may take place on a continuous basis, in parallel with the monitoring **1030,** or intermittently, but must be in either case happen within a sufficient short delay of the capturing of the signals to ensure that the pre-event video data of interest is still present in the first memory when the occurrence of a target event is established. The analyzing **1040** may take place locally, on the same device that collects the signals, or by a remote apparatus, connected to the former device by a network ("in the cloud").

When a target event is detected, the pre-event data of interest and the data pertaining to the actual event are transferred **1050** to a more permanent storage, referred to as the second memory, by copying **1051** a then recent portion of the video images from the first memory to the second memory; and storing **1052** video images captured in a predetermined period subsequent to the detection in the second memory. This transfer may happen in a single copying operation, at or shortly after the end of the event, when both the pre-event data of interest and the data pertaining to the actual event are present in the first memory.

Optionally, the equipment may be configured so as to allow a manual triggering of the transfer **1050** to the second memory, independently of the detection of a target event.

Preferably, an audio signal is captured in conjunction with the video images, and the resulting audio data is stored along with the stored video data. Preferably, the monitored physiological signals are also stored along with the stored video data. Throughout this application, any references to storage of "video data" may be read as optionally including the corresponding audio data and the corresponding physiological signal data.

Where a mobile device is used for video capturing **1010** and initial storage **1020,** the second memory may also be comprised in the mobile device. The mobile device may be equipped with a separate memory component (e.g. a flash memory) for this purpose, or it may use a different segment of the same memory component that provides the first memory.

Alternatively or additionally, the second memory may be comprised in an apparatus separate from said mobile device, the mobile device being configured to transmit data to the apparatus via a data communication network.

In an alternative setup, the device that captures the video and that receives the physiological signals continuously transmits that data to an external apparatus (e.g., a server to which the former device is connected via a data communication network such as the internet), and the external apparatus performs the cyclic storage **1020,** the analysis **1040,** and the selective transfer **1050** to permanent storage. Other functional divisions of the steps of the method according to the present invention between different devices are possible without deviating from the scope of the invention.

In a further variant of the invention, a low-quality video stream is stored in its entirety, and the selective event-centric storage described above is applied to a high-quality video stream.

The present invention also pertains to a computer program product comprising code means configured to cause a processor in a system with appropriate network interfaces, when executed, to perform the steps of the method described above. The computer program product may comprise computer code stored on a computer-readable medium such as, but not limited to, a magnetic carrier, an optical carrier, or semiconductor memory.

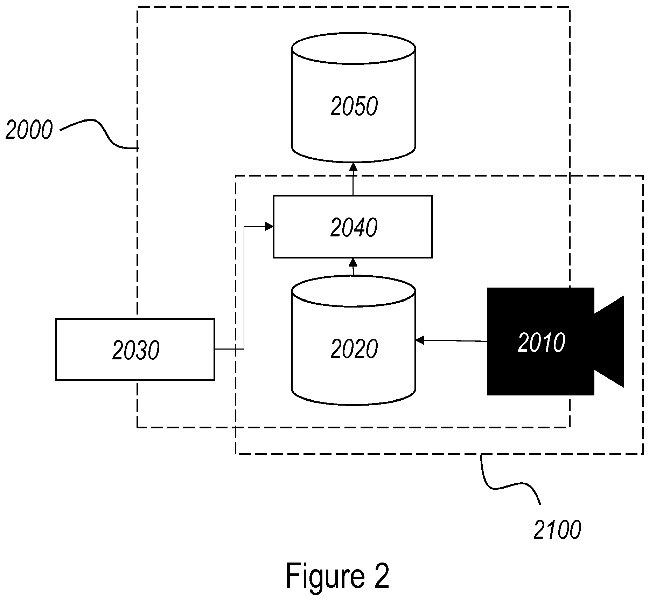

Figure 2 schematically illustrates an embodiment of the system according to the present invention.

The system **2000** comprises a camera **2010** arranged for capturing video images of the subject; a first memory **2020** adapted to cyclically store a recent portion of the captured video images; and sensors **2030** adapted to monitor one or more physiological signals of the subject.

The system **2000** further comprises a second memory **2050** adapted for long-term storage of video images.

The system **2000** comprises a processing means **2040** configured to:
o analyze said monitored physiological signals to allow detection of one or more target events; and
o upon detection of a target event:
   ▪ copy a then recent portion of said video images from said first memory **2020** to said second memory **2050;** and
   ▪ store video images captured in a predetermined period subsequent to said detection in said second memory **2050.**

The processing means may be implemented in dedicated hardware (e.g., ASIC), configurable hardware (e.g., FPGA), programmable components (e.g., a DSP or general purpose processor with appropriate software), or any combination thereof. The same component may also include other functions.

As explained above with reference to Figure 1, at least the camera **2010,** the first memory **2020,** and the processing means **2040** may be comprised in a mobile device.

Optional features described hereinabove with reference to embodiments of the method according to the present invention may be applied with the same technical effects and advantages to embodiments of the computer program product or the system of the present invention.

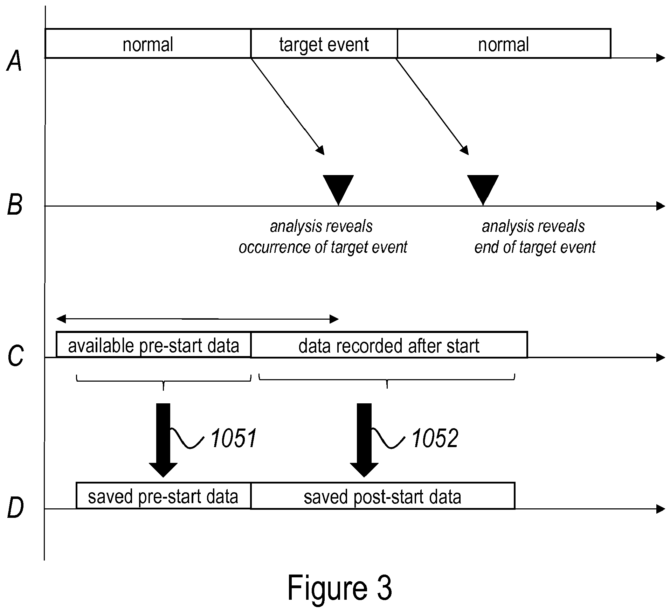

Figure 3 illustrates certain aspects of embodiments of the present invention by means of a schematic timing diagram. The four illustrated time axes, labeled A, B, C, and D, share a common time origin (illustrated by the vertical line joining the axes).

Axis A, which represents the monitored physiological parameters, illustrates the occurrence in time of a target event, between periods of normal readings.

Axis B, which illustrates the analysis of the monitored parameters, shows that the detection of the occurrence of the target event takes place shortly after the onset of the target event, and likewise, the detection of the end of the target event takes place shortly after the actual ending of the target event.

Axis C illustrates the content of the first memory (cyclic memory or short-term memory), and indicates the amount of "history", in terms of high-bandwidth data (in particular video data), available in the first memory at the time of detection of the target event (double arrow).

Axis D represents the second memory (long-term memory). In the illustrated case, a recent portion of the high-bandwidth data is transferred **1051** to the second memory, whereby "recent" is defined relative to the detected start time of the target event. In addition, a certain amount of high-bandwidth data captured after the event detection is copied to the second memory **1052** (or recorded directly thereto). In this scenario, the amount of time spanned by the post-detection data to be transferred **1052** must take into account the duration of the target event; it may in particular be useful to include some data pertaining to a certain amount of time after the end of the target event in the transfer **1052** to the second memory. As can be seen from this diagram, the dimensioning of the first memory must take into account the time offset between actual start of a target event and the detection of the target event.

While the invention has been described hereinabove with reference to specific embodiments, this was done to clarify and not to limit the invention, the scope of which is to be determined by reference to the accompanying claims.

## Claims

1. A method for monitoring a subject, the method comprising:
- capturing (1010) high-bandwidth data pertaining to said subject;
- cyclically storing (1020) a recent portion of said high-bandwidth data in a first memory (2020);
- monitoring (1030) one or more physiological signals of said subject;
- analyzing (1040) said monitored physiological signals to allow detection of one or more target events;
- upon detection of a target event:
o copying (1051) a then recent portion of said high-bandwidth data from said first memory (2020) to a second memory (2050); and
o storing (1052) high-bandwidth captured in a predetermined period subsequent to said detection in said second memory (2050).

2. The method according to claim 1, wherein said high-bandwidth data comprises video images of said subject.

3. The method according to claim 1 or claim 2, wherein said high-bandwidth data comprises high-resolution EEG signals.

4. The method according to any of the preceding claims, wherein said storing (1052) of said high-bandwidth data captured in said predetermined period subsequent to said detection comprises copying a further portion of said high-bandwidth data from said first memory (2020) to said second memory (2050).

5. The method according to any of the preceding claims, wherein said first memory (2020) is comprised in a mobile device (2100) and said capturing (1010) of said high-bandwidth data is performed using a sensor (2010) associated with said mobile device (2100).

6. The method according to claim 6, wherein said second memory (2050) is comprised in said mobile device.

7. The method according to claim 6, wherein said second memory (2050) is comprised in an apparatus separate from said mobile device (2100), said mobile device (2100) being configured to transmit data to said apparatus via a data communication network.

8. The method according to any of claims 5-7, wherein said high-bandwidth data comprises video images and said sensor associated with said mobile device (2100) is a camera (2010) integrated in said mobile device (2100).

9. The method according to any of the preceding claims, wherein said one or more physiological signals include one or more of the group of EEG, ECG, body temperature, movement, skin conductivity, heart rate, oxygen saturation.

10. The method according to any of the preceding claims, wherein said one or more target events include epileptic seizures.

11. The method according to any of the preceding claims, wherein said analyzing comprises applying a deep-learning algorithm.

12. A computer program product comprising code means configured to cause a processor, when executed, to perform the steps of the method according to any of the preceding claims.

13. A system for monitoring a subject, the system comprising:
- a first sensor (2010) arranged for capturing (1010) high-bandwidth data pertaining to said subject;
- a first memory (2020) adapted to cyclically store a recent portion of said captured high-bandwidth data;
- a second memory (2050) adapted for long-term storage of high-bandwidth data;
- second sensors (2030) adapted to monitor (1030) one or more physiological signals of said subject; and
- processing means (2040) configured to:
o analyze (1040) said monitored physiological signals to allow detection of one or more target events; and
o upon detection of a target event:
▪ copy a then recent portion of said high-bandwidth data from said first memory (2020) to said second memory (2050); and
▪ store high-bandwidth data captured in a predetermined period subsequent to said detection in said second memory (2050).

14. The system according to claim 13, wherein said high-bandwidth data comprises video images and wherein said first sensor (2010) arranged for capturing (1010) high-bandwidth data pertaining to said subject comprises a camera arranged for capturing video images of said subject.

15. The system according to claim 14, wherein at least said camera (2010), said first memory (2020), and said processing means (2040) are comprised in a mobile device (2100).
